# EUROPEAN PATENT SPECIFICATION

(11) **EP 3 985 094 B1**
(45) Date of publication and mention of the grant of the patent: **02.08.2023**
(21) Application number: 21192420.4
(22) Date of filing: 20.08.2021
(51) Int. Cl.: C12M 1/107, B01D 53/14, B01D 53/52, C10L 3/10, C12M 1/00

(54) **SYSTEM AND PROCESS FOR BIOGAS UPGRADING**
SYSTEM UND VERFAHREN ZUR VEREDELUNG VON BIOGAS
SYSTÈME ET PROCÉDÉ DE VALORISATION DU BIOGAZ

(30) Priority: 14.10.2020 BE 202005718
(43) Date of publication of application: 20.04.2022
(73) Proprietor: B.A.T. SERVICES bvba, 9051 Sint-Denijs-Westrem (BE)
(72) Inventor: De Lathauwer, Bart, 9051 Sint-Denijs-Westrem (BE)
(74) Representative: De Clercq & Partners

(56) References cited:
- EP-A1- 2 543 426
- DE-B- 1 083 015
- DE-U1- 20 300 663
- US-A- 5 480 524
- US-A1- 2010 107 872
- US-B1- 7 220 391

## Description

### TECHNICAL FIELD

The present invention relates to a system and process for upgrading biogas and other methane containing gas mixtures to a methane-rich gas. In particular, it concerns an improved upgrading system with water scrubber technology that is integrated with an UV/titanium dioxide-based system for water treatment that mainly removes methane slip and hydrogen sulphide.

### BACKGROUND

Biogas is produced by the anaerobic digestion or fermentation of wastewaters and solid residues. The material digested can include organic matter such as manure, sewage sludge, biodegradable waste or any other biodegradable feedstock. The process is essentially the degradation of organic compounds in the absence of oxygen to produce a saturated gas mixture mainly of methane and carbon dioxide and little hydrogen sulphide and other undesired substances.

Before biogas can be used as e.g. fuel for vehicles it must be "upgraded" or enriched in methane to become bio-methane. The purpose of biogas upgrading is to obtain a methane-rich product gas by separating mainly carbon dioxide, but also water and hydrogen sulphide, from the biogas.

One of the most used technologies for biogas upgrading is water scrubbing. Biogas is usually compressed to 6-8 bar with the water scrubber technology and led to an absorption column where it meets a counter flow of (process or wash) water. Due to the difference in solubility, carbon dioxide is dissolved in water and thereby separated from the upgraded biogas (enriched in methane) that can be extracted from the top of the absorption column. Also other components of the biogas, including hydrogen sulphide and traces of methane are absorbed into the water. The water is regenerated by a first pressure reduction in a flash column from which a part of carbon dioxide and methane dissolved in the water goes back to the incoming biogas flow. Then, the water that still contains CO₂ and trace amounts of CH₄ and H₂S, is led to a desorption column where mainly carbon dioxide is discharged from the water with e.g. air stripping. Besides, off-gases also contain methane and hydrogen sulphide. Such system for upgrading a methane containing biogas mixture comprising an absorption column, a flash column and a desorption column has been described e.g. in DE20300663. The system of DE20300663 does not comprise an UV irradiation water treatment system that is integrated between the flash column and the desorption column.

Since methane is a strong greenhouse gas, having a global warming potential about 30 times higher than carbon dioxide, the release of methane to the atmosphere should be minimized. Also to comply with emission regulations, the amount of the noxious compound hydrogen sulphide that is released from the water scrubber should be kept as low as possible.

Accordingly, there is a need for improved water scrubbers wherein the amount of methane and/or hydrogen sulphide that leaves the water scrubber with the off-gas is kept to a minimum.

### SUMMARY

The present invention relates to a system for biogas upgrading by means of water scrubbing that is integrated with a system for water treatment by ultraviolet irradiation combined with photocatalysis such as titanium dioxide-mediated photocatalysis. In particular, an UV irradiation water treatment system is integrated in the water scrubber between the flash column and the desorption column, said UV irradiation water treatment system comprising a housing encasing one or more UV lamps that are surrounded by a protective tube. The present inventors have found that oxidation of methane and/or hydrogen sulphide can be substantially improved upon covering the outer side of the tube(s) surrounding the UV lamp(s) with a net body onto which photocatalyst such as titanium dioxide is attached.

The present invention is in particular captured by any one or any combination of one or more of the below numbered aspects and embodiments (i) to (x) wherein:
(i) A system (100) for upgrading a methane containing gas mixture comprising:
   an absorption column (110) for absorbing CO₂ and H₂S and a small amount of CH₄ from the gas mixture (1, 2) into water;
   a flash column (120) for liberating at least a portion of the gases (4) absorbed in the absorption column from the water (10), and
   a desorption column (130) for removing dissolved gases (6) from the water (12) by (air) stripping,
   wherein at least a portion of the desorbed water (13) is returned to the absorption column (110),
   characterized in that an UV irradiation water treatment system (140) is integrated between the flash column (120) and the desorption column (130), said UV irradiation water treatment system (140) comprising a housing encasing one or more ultraviolet lamps which are surrounded by a protective tube, wherein the outer side of said protective tube(s) surrounding the one or more ultraviolet lamps is covered with a net body and photo-catalyst attached on said net body and said housing having a water inlet and a water outlet,
   wherein said absorption column (110), said flash column (120), said UV irradiation water treatment system (140) and said desorption column (130) are in fluid connection.
(ii) The system according to (i), wherein the net body is a metal net.
(iii) The system according to (i) or (ii), wherein the photo-catalyst is titanium dioxide, preferably titantium dioxide in the anastase form.
(iv) The system according to any one of (i) to (iii), wherein the one or more ultraviolet lamps are arranged substantially parallel to the water flow in the housing.
(v) A process for upgrading a methane containing gas mixture (1) comprising:
   (a) contacting the gas mixture (1, 2) in an absorption column (110) with water (13) to effect absorption of CO₂ and H₂S and a small amount of CH₄,
   (b) passing the water (10) containing the absorbed gases to a flash column (120) maintained at a pressure substantially lower than in the absorption column (110) to effect liberation of at least a portion of the absorbed gases (4),
   (c) withdrawing from said flash column (120) gases (4) liberated therein and returning said gases (4) to the absorption column (110),
   (d) withdrawing water (11) containing CO₂, H₂S and CH₄ from said flash column (120),
   (e) introducing said water (11) containing CO₂, H₂S and CH₄ into an UV irradiation water treatment system (140) to effect oxidative degradation of CH₄ and/or H₂S, wherein said UV irradiation water treatment system (140) comprises a housing encasing one or more ultraviolet lamps which are surrounded by a protective tube, wherein the outer side of said protective tube(s) surrounding the one or more ultraviolet lamps is covered with a net body and photo-catalyst attached on said net body and said housing having a water inlet and a water outlet,
   (f) withdrawing the water (12) from the UV irradiation system (140),
   (g) introducing the water (12) withdrawn from the UV irradiation system (140) to a desorption column (130) to effect removal of substantially all of the dissolved gases (6) by stripping, and
   (h) returning at least a portion of the desorbed water (13) to the absorption column (110),
   wherein a system (100) according to any one of (i) to (iv) is used.
(vi) The process according to (v), wherein said oxidative degradation of CH₄ and/or H₂S is by photochemical oxidation
(vii) The process according to (v) or (vi), wherein said oxidative degradation of CH₄ and/or H₂S is by photochemical oxidation and photocatalytic oxidation.
(viii) The process according to (vii), wherein said photocatalytic oxidation of CH₄ and/or H₂S is mediated by titanium dioxide.
(ix) The process according to any one of (v) to (viii), wherein said stripping is air (5) stripping.
(x). The process according to any one of (v) to (ix), wherein said gas mixture (1) is biogas.

### DESCRIPTION OF THE FIGURES

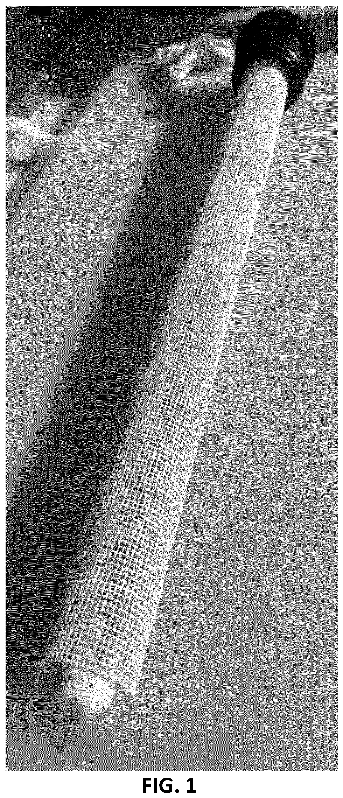

**Fig. 1** shows an ultraviolet lamp surrounded by a protective quartz glass tube, wherein the outer side of the quartz glass tube is covered with a metal net onto which titanium dioxide in anastase form is attached.
**Fig. 2** shows a schematic of a system (100) and method according to an embodiment of the invention.

### DETAILED DESCRIPTION

Before the present processes and systems according to the invention are described, it must be understood that the invention is not limited to the particular processes and systems described, as such processes and systems can naturally vary. It must also be clear that the terminology used herein is not intended to be limiting, as the scope of the present invention can only be limited by the relevant claims.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims and statements, any of the embodiments can be used in any combination.

Although all methods and materials that are the same or equivalent to those described herein may be used in practice or in testing of the present invention, the preferred methods and materials will now be described.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

As used herein, the singular forms "a," "an" and "the" include both singular and plural referents, unless the context clearly indicates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein. This applies to numerical ranges irrespective of whether they are introduced by the expression "from... to..." or the expression "between... and..." or another expression.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

### Water scrubber

The present invention relates to a system (100) and process for upgrading a methane containing gas mixture by means of water scrubbing, wherein a system for water treatment by ultraviolet irradiation is integrated. Any conventional water scrubbing system known to the skilled person can be used in the present invention and can be used under conventional operating conditions. A typical water scrubber comprises an absorption column, a flash column and a desorption column, which are in fluid connection with each other.

Water scrubbing involves passing the gas stream through an absorption column (also referred to herein as absorber or scrubber) (110). Raw gas (1, 2) enters the absorption column at the bottom whereas water (13) is fed at the top thereof and so the absorption process is operated in counter-current. The absorption column may be provided with random packing to provide a high contact surface area at a gas/water interface in order to obtain maximum mass transfer. The scrubber operates at an elevated pressure to force gas absorption into water; the gas may be compressed in a compressor (150) up to 6-8 bars. The scrubber can operate at temperatures between just above 0°C - 20°C, such as between 2°C - 10°C. In the absorber, components of the gas, mainly CO₂, but also traces of CH₄ and H₂S, are absorbed into the water until saturation equilibrium is reached. The gas (3) leaving the absorption column is enriched with methane, typically the gas leaving the absorption column has a methane concentration from 70 to 98% by volume, depending on the initial gas composition and quality. However, it also contains residual traces of hydrogen sulphide and carbon dioxide. This discharged gas is also saturated with water. Approximate gas contents can be: 97% - 99% methane, 1% - 2.5% carbon dioxide, 0.5% insoluble contaminants (oxygen and nitrogen from the air dissolved in the scrubbing water or air contamination of the raw gas stream), 5 PPM hydrogen sulphide, water vapour.

Since methane is partly water soluble, the water (10) from the absorption column (110) is conveyed to a flash column (120) in order to lower the methane losses. The water is depressurized in the flash column down to between 2 and 3 bar and at least a part of the dissolved gas (4) comes out. The dissolved gas (4) , which contains some methane but mainly carbon dioxide, is at least partially released and transferred back to the raw gas (1) inlet.

The water (11) containing the absorbed carbon dioxide and/or hydrogen sulphide, but also some methane, which is leaving the flash column (120) can be regenerated and recirculated back to the absorption column. The regeneration may be carried out by stripping, in particular air (5) stripping, in a desorption column (130), which is similar to the packed absorption column to obtain a large mass transfer efficiency.

### UV irradiation water treatment system

The systems (100) and processes described herein are characterized in that an UV irradiation water treatment system (140) is integrated between the flash column (120) and desorption column (130) of the water scrubber. In particular, a water outlet of the flash column is in fluid connection with a water inlet of the UV irradiation system, and a water outlet of the UV irradiation system is in fluid connection with a water inlet of the desorption column.

An UV irradiation water treatment system (140) as used herein comprises a housing encasing one or more UV lamps which are surrounded by a tube, said housing having a water inlet for allowing water to enter the housing and a water outlet for allowing water to leave the housing. The UV lamp(s) is(are) electrically connected to an electrical power source. In embodiments, the UV lamp(s) has a power of 24 Watt or less. In particular embodiments, the UV lamp(s) has a power of about 24 Watt. In embodiments, the UV lamp(s) emit UV light having a wavelength between about 200 nm and about 315 nm, preferably between about 200 nm and about 280 nm. In particular embodiments, the UV lamp(s) emits UV light having a wavelength of about 254 nm.

The UV lamps are surrounded by a protective tube; the water flowing past the tubing in the housing, i.e. a water passage is formed bounded by at least an interior surface of the housing and an exterior surface of the tube surrounding the UV lamp. The tube itself is preferably constructed of a non-UV-absorbing material, such as, e.g., quartz glass, high-silica glass or borosilicate glass. In preferred embodiments, the tube is a quartz glass tube.

The UV irradiation system may comprise one UV lamp surrounded by a tube. The system may also comprise two or more UV lamps, each surrounded by a tube. Said two or more UV lamps are preferably arranged in parallel in the housing.

Preferably, the UV lamp(s) is arranged parallel to the water flow in the housing in order to maximize the amount of time in which the water is exposed to UV radiation.

The housing forms a passage to direct water along a path inside the housing. The housing can have any shape, preferably the housing has a tubular shape. The housing is preferably watertight, so that water cannot escape from the housing except through the inlet and outlet. The housing may be made, or include, a material sufficient to prevent UV radiation from escaping the housing. For example, the housing may be made from stainless steel.

In embodiments, the interior surface of the housing is made of, includes or is coated with a material that is reflective to UV light, to increase UV exposure.

The herein described UV irradiation systems for water treatment are known for the disinfection of water and any such system can be used for the purposes of the present invention. Non-limiting examples include the Spektron UV series and the Aquada UV systems of Wedeco. Accordingly, in embodiments, the UV irradiation water treatment system is an UV irradiation system capable of disinfecting water.

The present inventors have found that an UV irradiation water treatment system (140) as described herein allows for the oxidative degeneration of methane and/or hydrogen sulphide absorbed in the water (11). The process of UV-activated oxidation is based on natural processes. In particular, organic substances are excited under the action of ultraviolet rays in such a way that, in the presence of a suitable oxidizing agent, oxidative degradation takes place. Without wishing to be bound by theory, non-limiting examples of oxidation reactions that can occur in the UV irradiation water treatment system include:

CH₄ + 2 O₂ → CO₂ + 2 H₂O (total oxidation)

CH₄ + 0.5 O₂ → CO + 2 H₂ (partial oxidation)

CO + 0.5 O₂ → CO₂ (CO oxidation)

H₂ + 0.5 O₂ → H₂O (hydrogen oxidation)

CH₄ + H₂O → CO + 3 H₂ (steam reforming)

CH₄ + CO₂ → 2 CO + 2 H₂ (CO₂ (dry) reforming)

CO + H₂O → CO₂ + H₂ (water gas shift)

2 H₂S + 3 O₂ → 2 SO₄ + 2 H₂O

As a result of the oxidative degradation of methane and/or hydrogen sulphide in the UV irradiation system, the water (12) leaving the UV irradiation system (140) has a reduced methane and/or hydrogen sulphide content. Consequently, water (12) with a reduced methane and/or hydrogen sulphide content enters the desorption column (130) of the water scrubber system, resulting in less methane and/or hydrogen sulphide leaving the water scrubber system with the off-gas (6).

The oxidative degradation of methane and/or hydrogen sulphide was found to be substantially improved upon covering the tube surrounding the UV lamp with a net body onto which a photocatalyst was attached allowing for both: photochemical and photocatalytic methane oxidation reactions.

Accordingly, the outer side of the tube(s) surrounding the UV lamp(s) is covered with a net body onto which a photocatalyst is attached.

By using a net body, UV irradiation can still pass the tubing and irradiate the water flowing past the tubing, allowing for both photochemical and photocatalytic oxidation reactions. In particular embodiments, the net body is a metal net.

The photocatalyst is an UV-activated photocatalyst and may include photo-activated semiconductors such as titanium dioxide (TiO₂) (photo activation wavelength; not more than 388 nm), tungsten oxide; WO₂ (photo activation wavelength; not more than 388 nm), zinc oxide; ZnO (photo activation wavelength; not more than 388 nm), zinc sulfide; ZnS (photo activation wavelength; not more than 344 nm) and tin oxide; SnO₂ (photo activation wavelength; not more than 326 nm).

In particular embodiments, the photocatalyst that is attached to the net body is titanium dioxide (TiO₂), preferably titanium dioxide in the anastase form. Titanium dioxide may be attached to the net body using a TiO₂-containing paint (e.g. StoColor Photosan, Sto).

As a result of the oxidative degradation of methane in the UV irradiation system (140), less methane and/or hydrogen sulphide are dissolved in the water (12) entering the desorption column (130) of the water scrubber system. Accordingly, the off-gas or stripping air (6) contains less methane and/or hydrogen sulphide compared to conventional water scrubbers.

### Process

A method or process for upgrading biogas or another methane containing gas mixture (1) using the herein described system is also provided. A gas (3) with an enriched content of methane remains after upgrading the biogas or other methane containing gas mixture (1) according to the process described herein. This upgraded gas remains unsuitable for immediate use due to its water saturation, and is usually subjected to a dehydration process to remove water. Dehydration processes are known to the skilled person and may include, without limitation, use of molecular sieve or porous materials.

The process provided herein includes the steps of:
(a) contacting the gas mixture (1, 2) in an absorption column (110) with water (13) to effect absorption of CO₂ and H₂S and a small amount of CH₄,
(b) passing the water (10) containing the absorbed gases to a flash column (120) maintained at a pressure substantially lower than in the absorption column to effect liberation of at least a portion of the absorbed gases (4),
(c) withdrawing from said flash column gases (4) liberated therein and returning said gases to the absorption column (110),
(d) withdrawing water (11) containing CO₂, H₂S and CH₄ from said flash column (120),
(e) introducing said water (11) containing CO₂, H₂S and CH₄ into an UV irradiation water treatment system (140) to effect oxidative degradation of CH₄ and/or H₂S, wherein said UV irradiation water treatment system (140) comprises a housing encasing one or more ultraviolet lamps which are surrounded by a protective tube, wherein the outer side of said protective tube(s) surrounding the one or more ultraviolet lamps is covered with a net body and photo-catalyst attached on said net body and said housing having a water inlet and a water outlet,
(f) withdrawing the water (12) from the UV irradiation system (140),
(g) introducing the water (12) withdrawn from the UV irradiation system (140) to a desorption column (130) to effect removal of substantially all of the dissolved gases (6) by (air) stripping, and
(h) returning at least a portion of the desorbed water (13) to the absorption column (110).

As described elsewhere herein, upon passing water (11) containing CO₂, H₂S and CH₄ through an UV irradiation system (140) as described herein, UV-activated oxidation of methane and/or hydrogen sulphide (i.e. photochemical oxidation) takes place. As a result, water (12) with a reduced methane and/or hydrogen sulphide content enters the desorption column (130) and less methane will be released with the off-gas or stripping air (6).

Oxidative degradation of CH₄ and/or H₂S is by a combination of photochemical oxidation and photocatalytic oxidation, resulting in a further reduction of the methane and/or hydrogen sulphide content. In particular embodiments, photocatalytic oxidation of methane and/or hydrogen sulphide is mediated by titanium dioxide.

As shown herein, photochemical and photocatalytic oxidation of methane and/or hydrogen sulphide is achieved by covering the outer side of a protective tube(s) surrounding an UV lamp with a net body such as a metal net, and attaching a photocatalyst onto said net body. Optionally, the inner side of the housing may be coated with a photocatalyst coating, e.g. titanium dioxide. Such UV irradiation systems are known to the skilled person and include, for example, the titanium Advanced Oxidation Process (AOP) system of Brightwater.

The systems and processes described herein are particularly useful for upgrading biogas. Accordingly, in preferred embodiments, the methane containing gas mixture is biogas.

### EXAMPLES

### Example 1: Water scrubber system with integrated UV irradiation water treatment system

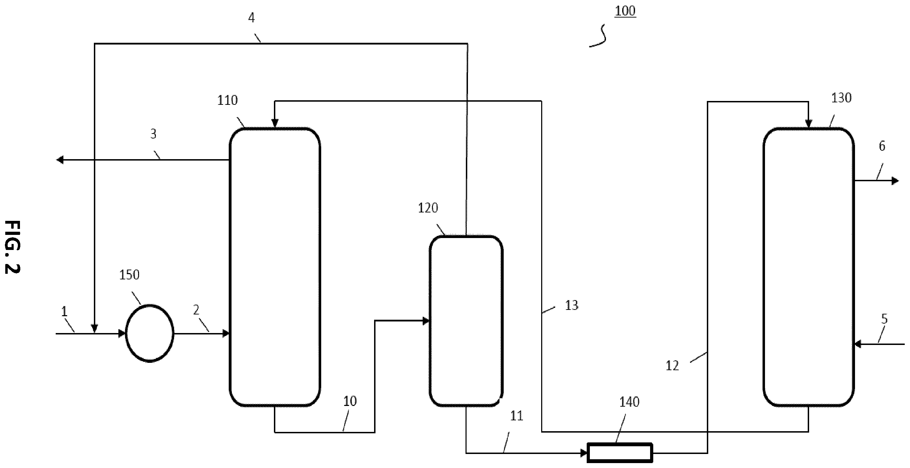

An embodiment of the invention is discussed with reference to Fig. 2. Biogas stream 1 in Figure 2 is merged with gas stream 4 and is led into a compressor 150 to raise the pressure of the gas stream. The compressed gas stream 2 is led to an absorption column 110 where it meets a counter flow of water. Components of the gas, mainly CO₂, but also H₂S and traces of CH₄, are absorbed into the water until saturation equilibrium is reached. The gas stream 3 leaving the absorption column 110 is enriched with methane. The water stream 10 from the absorption column 110 containing the absorbed gases is conveyed to a flash column 120. The water is depressurized in the flash column 120; the absorbed gases are thereby partially released. This gas stream 4 from the flash column 120, which mainly contains CH₄ and CO₂, is returned to biogas stream 1. The water stream 11 leaving the flash column 120 is introduced into an UV irradiation water treatment system 140 to effect oxidative degradation of methane and hydrogen sulphide that is still dissolved in the water by photochemical oxidation optionally combined with photocatalytic oxidation. The water stream 12 that leaves the UV irradiation system 140 is introduced into a desorption column 130. Dissolved gases in the water stream 12 are released by air 5 stripping via an off-gas stream 6. This off-gas stream 6 mainly contains CO₂, and has a reduced methane and H₂S content due to the oxidative degradation of methane and H₂S in the UV irradiation system. The desorbed water stream 13 leaving the desorption column 130 is returned to the absorption column 110.

## Claims

1. A system (100) for upgrading a methane containing gas mixture comprising:
an absorption column (110) for absorbing CO₂ and H₂S and a small amount of CH₄ from the gas mixture (1, 2) into water;
a flash column (120) for liberating at least a portion of the gases (4) absorbed in the absorption column from the water (10), and
a desorption column (130) for removing dissolved gases (6) from the water (12) by (air) stripping,
wherein at least a portion of the desorbed water (13) is returned to the absorption column (110),
**characterized in that** an UV irradiation water treatment system (140) is integrated between the flash column (120) and the desorption column (130), said UV irradiation water treatment system (140) comprising a housing encasing one or more ultraviolet lamps which are surrounded by a protective tube, wherein the outer side of said protective tube(s) surrounding the one or more ultraviolet lamps is covered with a net body and photo-catalyst attached on said net body and said housing having a water inlet and a water outlet,
wherein said absorption column (110), said flash column (120), said UV irradiation water treatment system (140) and said desorption column (130) are in fluid connection.

2. The system according to claim 1, wherein the net body is a metal net.

3. The system according to claim 1 or 2, wherein the photo-catalyst is titanium dioxide, preferably titantium dioxide in the anastase form.

4. The system according to any one of claims 1 to 3, wherein the one or more ultraviolet lamps are arranged substantially parallel to the water flow in the housing.

5. A process for upgrading a methane containing gas mixture (1) comprising:
(a) contacting the gas mixture (1, 2) in an absorption column (110) with water (13) to effect absorption of CO₂ and H₂S and a small amount of CH₄,
(b) passing the water (10) containing the absorbed gases to a flash column (120) maintained at a pressure substantially lower than in the absorption column (110) to effect liberation of at least a portion of the absorbed gases (4),
(c) withdrawing from said flash column (120) gases (4) liberated therein and returning said gases (4) to the absorption column (110),
(d) withdrawing water (11) containing CO₂, H₂S and CH₄ from said flash column (120),
(e) introducing said water (11) containing CO₂, H₂S and CH₄ into an UV irradiation water treatment system (140) to effect oxidative degradation of CH₄ and/or H₂S, wherein said UV irradiation water treatment system (140) comprises a housing encasing one or more ultraviolet lamps which are surrounded by a protective tube, wherein the outer side of said protective tube(s) surrounding the one or more ultraviolet lamps is covered with a net body and photo-catalyst attached on said net body and said housing having a water inlet and a water outlet,
(f) withdrawing the water (12) from the UV irradiation system (140),
(g) introducing the water (12) withdrawn from the UV irradiation system (140) to a desorption column (130) to effect removal of substantially all of the dissolved gases (6) by stripping, and
(h) returning at least a portion of the desorbed water (13) to the absorption column (110), wherein a system (100) according to any one of claims 1 to 4 is used.

6. The process according to claim 4, wherein said oxidative degradation of CH₄ and/or H₂S is by photochemical oxidation

7. The process according to claim 5 or 6, wherein said oxidative degradation of CH₄ and/or H₂S is by photochemical oxidation and photocatalytic oxidation.

8. The process according to claim 7, wherein said photocatalytic oxidation of CH₄ and/or H₂S is mediated by titanium dioxide.

9. The process according to any one of claims 5 to 8, wherein said stripping is air (5) stripping.

10. The process according to any one of claims 5 to 9, wherein said gas mixture (1) is biogas.

## Patentansprüche

1. System (100) zum Aufwerten eines methanhaltigen Gasgemischs, umfassend:
eine Absorptionskolonne (110) zum Absorbieren von CO₂ und H₂S und einer kleinen Menge CH₄ aus dem Gasgemisch (1, 2) in Wasser;
eine Flash-Kolonne (120) zum Freisetzen wenigstens eines Teils der in der Absorptionskolonne (110) absorbierten Gase (4) aus dem Wasser (10) und
eine Desorptionskolonne (130) zum Entfernen von gelösten Gasen (6) aus dem Wasser durch (Luft)strippen,
wobei wenigstens ein Teil des desorbierten Wassers (13) zu der Absorptionskolonne (110) zurückgeführt wird,
**dadurch gekennzeichnet, dass** ein UV-Bestrahlungs-Wasserbehandlungssystem (140) zwischen die Flash-Kolonne (120) und die Desorptionskolonne (130) eingegliedert ist, wobei das UV-Bestrahlungs-Wasserbehandlungssystem (140) ein Gehäuse umfasst, das eine oder mehrere Ultraviolettlampen einschließt, die von einem Schutzrohr umgeben sind, wobei die Außenseite des Schutzrohrs bzw. der Schutzrohre, das/die die eine oder mehreren Ultraviolettlampen umgibt/umgeben, von einem Netzkörper und an dem Netzkörper angebrachtem Photokatalysator bedeckt ist/sind, und das Gehäuse einen Wassereinlass und einen Wasserauslass aufweist,
wobei sich die Absorptionskolonne (110), die Flash-Kolonne (120), das UV-Bestrahlungs-Wasserbehandlungssystem (140) und die Desorptionskolonne (130) in Fluidverbindung befinden.

2. System gemäß Anspruch 1, wobei der Netzkörper ein Metallnetz ist.

3. System gemäß Anspruch 1 oder 2, wobei der Photokatalysator Titandioxid, vorzugsweise Titandioxid in der Anastaseform, ist.

4. System gemäß einem der Ansprüche 1 bis 3, wobei die eine oder mehreren Ultraviolettlampen im Wesentlichen parallel zu dem Wasserstrom in dem Gehäuse angeordnet sind.

5. Verfahren zum Aufwerten eines methanhaltigen Gasgemischs (1), umfassend:
(a) Inkontaktbringen des Gasgemischs (1, 2) in einer Absorptionskolonne (110) mit Wasser (13), um Absorption von CO₂ und H₂S und einer kleinen Menge CH₄ zu bewirken;
(b) Leiten des Wassers (10), das die absorbierten Gase enthält, zu einer Flash-Kolonne (120), die bei einem Druck von wesentlich niedriger als jener in der Absorptionskolonne (110) gehalten wird, um Freisetzen wenigstens eines Teils der absorbierten Gase (4) zu bewirken,
(c) Entnehmen von darin freigesetzten Gasen (4) aus der Flash-Kolonne (120) und Rückführen der Gase (4) zu der Absorptionskolonne (110),
(d) Entnehmen von Wasser (11), das CO₂, H₂S und CH₄ enthält, aus der Flash-Kolonne (120),
(e) Einführen des Wassers (11), das CO₂, H₂S und CH₄ enthält, in ein UV-Bestrahlungs-Wasserbehandlungssystem (140), um oxidativen Abbau von CH₄ und/oder H₂S zu bewirken, wobei das UV-Bestrahlungs-Wasserbehandlungssystem (140) ein Gehäuse umfasst, das eine oder mehrere Ultraviolettlampen einschließt, die von einem Schutzrohr umgeben sind, wobei die Außenseite des Schutzrohrs bzw. der Schutzrohre, das/die die eine oder mehreren Ultraviolettlampen umgibt/umgeben, von einem Netzkörper und an dem Netzkörper angebrachtem Photokatalysator bedeckt ist/sind, und das Gehäuse einen Wassereinlass und einen Wasserauslass aufweist,
(f) Entnehmen des Wassers (12) aus dem UV-Bestrahlungssystem (140),
(g) Einführen des aus dem UV-Bestrahlungssystem (140) entnommenen Wassers (12) in eine Desorptionskolonne (130), um Entfernen von im Wesentlichen der gesamten gelösten Gase (6) durch Strippen zu bewirken, und
(h) Rückführen wenigstens eines Teils des desorbierten Wassers (13) zu der Absorptionskolonne (110),
wobei ein System (100) gemäß einem der Ansprüche 1 bis 4 verwendet wird.

6. Verfahren gemäß Anspruch 4, wobei der oxidative Abbau von CH₄ und/oder H₂S durch photochemische Oxidation erfolgt.

7. Verfahren gemäß Anspruch 5 oder 6, wobei der oxidative Abbau von CH₄ und/oder H₂S durch photochemische Oxidation und photokatalytische Oxidation erfolgt.

8. Verfahren gemäß Anspruch 7, wobei die photokatalytische Oxidation von CH₄ und/oder H₂S von Titandioxid vermittelt wird.

9. Verfahren gemäß einem der Ansprüche 5 bis 8, wobei das Strippen Luft(5)-Strippen ist.

10. Verfahren gemäß einem der Ansprüche 5 bis 9, wobei das Gasgemisch (1) Biogas ist.

## Revendications

1. Système (100) pour la valorisation d'un mélange de gaz contenant du méthane comprenant :
une colonne d'absorption (110) pour l'absorption de CO₂ et de H₂S et d'une petite quantité de CH₄ du mélange de gaz (1, 2) dans de l'eau ;
une colonne flash (120) pour libérer au moins une partie des gaz (4) absorbés dans la colonne d'absorption de l'eau (10), et
une colonne de désorption (130) pour éliminer des gaz dissous (6) de l'eau (12) par strippage (à l'air),
au moins une partie de l'eau désorbée (13) étant renvoyée vers la colonne d'absorption (110),
**caractérisé en ce qu'**un système de traitement de l'eau par irradiation UV (140) est intégré entre la colonne flash (120) et la colonne de désorption (130), ledit système de traitement de l'eau par irradiation UV (140) comprenant un boîtier renfermant une ou plusieurs lampes à ultraviolets qui sont entourées par un tube protecteur, le côté extérieur dudit ou desdits tubes protecteurs entourant la ou les lampes à ultraviolets étant recouvert d'un corps de filet et d'un photo-catalyseur fixé sur ledit corps de filet et ledit boîtier ayant une entrée d'eau et une sortie d'eau, ladite colonne d'absorption (110), ladite colonne flash (120), ledit système de traitement de l'eau par irradiation UV (140) et ladite colonne de désorption (130) étant en connexion fluidique.

2. Système selon la revendication 1, le corps de filet étant un filet de métal.

3. Système selon la revendication 1 ou 2, le photo-catalyseur étant du dioxyde de titane, préférablement du dioxyde de titane sous la forme anastase.

4. Système selon l'une quelconque des revendications 1 à 3, la ou les lampes à ultraviolets étant agencées sensiblement parallèlement au flux d'eau dans le boîtier.

5. Procédé pour la valorisation d'un mélange de gaz contenant du méthane (1) comprenant :
(a) la mise en contact du mélange de gaz (1, 2) dans une colonne d'absorption (110) avec de l'eau (13) pour effectuer l'absorption de CO₂ et de H₂S et d'une petite quantité de CH₄,
(b) le passage de l'eau (10) contenant les gaz absorbés vers une colonne flash (120) maintenue à une pression sensiblement inférieure à celle dans la colonne d'absorption (110) pour effectuer la libération d'au moins une partie des gaz absorbés (4),
(c) le soutirage de ladite colonne flash (120) de gaz (4) libérés dans celle-ci et renvoi desdits gaz (4) vers la colonne d'absorption (110),
(d) le soutirage d'eau (11) contenant CO₂, H₂S et CH₄ de ladite colonne flash (120),
(e) l'introduction de ladite eau (11) contenant CO₂, H₂S et CH₄ dans un système de traitement de l'eau par irradiation UV (140) pour effectuer la dégradation oxydante de CH₄ et/ou de H₂S, ledit système de traitement de l'eau par irradiation UV (140) comprenant un boîtier renfermant une ou plusieurs lampes à ultraviolets qui sont entourées par un tube protecteur, le côté extérieur dudit ou desdits tubes protecteurs entourant la ou les lampes à ultraviolets étant recouvert d'un corps de filet et d'un photo-catalyseur fixé sur ledit corps de filet et ledit boîtier ayant une entrée d'eau et une sortie d'eau,
(f) le soutirage de l'eau (12) du système d'irradiation UV (140),
(g) l'introduction de l'eau (12) soutirée du système d'irradiation UV (140) dans une colonne de désorption (130) pour effectuer l'élimination de sensiblement tous les gaz dissous (6) par strippage, et
(h) le renvoi d'au moins une partie de l'eau désorbée (13) vers la colonne d'absorption (110),
un système (100) selon l'une quelconque des revendications 1 à 4 étant utilisé.

6. Procédé selon la revendication 4, ladite dégradation oxydante de CH₄ et/ou de H₂S étant par oxydation photochimique.

7. Procédé selon la revendication 5 ou 6, ladite dégradation oxydante de CH₄ et/ou de H₂S étant par oxydation photochimique et oxydation photocatalytique.

8. Procédé selon la revendication 7, ladite oxydation photocatalytique de CH₄ et/ou de H₂S étant médiée par du dioxyde de titane.

9. Procédé selon l'une quelconque des revendications 5 à 8, ledit strippage étant un strippage à l'air (5).

10. Procédé selon l'une quelconque des revendications 5 à 9, ledit mélange de gaz (1) étant un biogaz.
